Europäisches Patentamt

European Patent Office

Office européen des brevets

(19)

(11) Veröffentlichungsnummer : **0 017 051**
**B1**

(12) **EUROPÄISCHE PATENTSCHRIFT**

(45) Veröffentlichungstag der Patentschrift :
07.04.82

(21) Anmeldenummer : 80101338.4

(22) Anmeldetag : 14.03.80

(51) Int. Cl.³ : **C 07 C 69/24, C 07 C 67/38**

(54) **Verfahren zur Herstellung von Alkylestern gesättigter aliphatischer Carbonsäuren.**

(30) Priorität : 29.03.79 DE 2912489

(43) Veröffentlichungstag der Anmeldung :
15.10.80 (Patentblatt 80/21)

(45) Bekanntmachung des Hinweises auf die Patenterteilung : 07.04.82 Patentblatt 82/14

(84) Benannte Vertragsstaaten :
**BE DE FR GB IT NL**

(56) Entgegenhaltungen :
**DE - A - 1 963 804**
**DE - A - 2 263 907**

(73) Patentinhaber : **CHEMISCHE WERKE HÜLS AG**
**Postfach 1320**
**D-4370 Marl 1 (DE)**

(72) Erfinder : **Hofmann, Peter, Dr.**
**Lipper Weg 193**
**D-4370 Marl (DE)**

Imprimerie Jouve, 18, rue St-Denis, 75001 Paris, France

## Verfahren zur Herstellung von Alkylestern gesättigter aliphatischer Carbonsäuren

Es ist bekannt, daß man durch Umsetzung von Olefinen mit Kohlenmonoxid und einer H-aciden Komponente, wie z.B. Wasser, Alkanol und Amin, in Gegenwart eines Katalysators, enthaltend ein Metall der 8. Nebengruppe des Periodischen Systems der Elemente und einen Promotor, Fettsäuren bzw. die entsprechenden Fettsäurederivate herstellen kann (J. Falbe, Synthesen mit Kohlenmonoxyd, Springer-Verlag, Berlin, Heidelberg, New York, 1967).

Diese als Hydrocarboxylierung bekannte Reaktion dient in erster Linie zur Herstellung von Fettsäureestern. Dabei setzt man in aller Regel $\alpha$-Olefine mit niederen Alkanolen ($C_1$ bis $C_8$) in Gegenwart cobalt- oder nickelhaltiger Katalysatoren um. Als besonders aktiv haben sich Katalysatorsysteme aus einer Cobaltverbindung und Pyridin bzw. einem nicht ortho-substituierten Pyridinderivat erwiesen. Diese Systeme zeichnen sich gleichzeitig dadurch aus, daß sie zu Reaktionsprodukten hoher Linearität führen. Auch wird durch sie die als Nebenreaktion ablaufende Hydroformylierung, die bekanntlich Aldehyde und Acetale liefert, weitgehend unterdrückt (US-PS 3 507 891, DE-OSS 16 18 156, 19 63 804 und 20 23 690).

Die in diesen Druckschriften beschriebenen Verfahren des Standes der Technik schließen zwar den Einsatz von Olefinen mit innenständiger Doppelbindung generell nicht aus, jedoch zeigen die angeführten Beispiele, daß unter den für $\alpha$-Olefine günstigen Bedingungen, für Olefine mit innenständiger Doppelbindung keine befriedigenden Ergebnisse erzielt werden. So werden entweder die Bildungsgeschwindigkeit und die Linearität der Ester stark verringert oder es wird nur eine unzureichende Selektivität erreicht.

Aufgabe der vorliegenden Erfindung war es daher, ein Verfahren zu entwickeln, das es gestattet, Monoolefine mit innenständiger Doppelbindung, vornehmlich Gemische überwiegend innenständige Doppelbindungen enthaltender aliphatischer Monoolefine, mit gleichen oder nahezu gleichen Ergebnissen bezüglich Raum-Zeit-Ausbeute, Linearität und Selektivität wie $\alpha$-Olefine umzusetzen.

Diese Aufgabe wurde durch die in den Ansprüche beschriebene Kombination verfahrenskritischer Maßnahmen gelöst.

Die beim erfindungsgemäßen Verfahren einsetzbaren aliphatischen Monoolefine mit innenständiger Doppelbindung werden im allgemeinen nach bekannten Verfahren des Standes der Technik aus Paraffinen durch Dehydrierung oder Chlorierung und anschließende Dehydrochlorierung der erhaltenen Chlorparaffine hergestellt. Bei diesen Verfahren werden in der Regel Paraffinschnitte, d.h. Gemische unterschiedlicher C-Zahl eingesetzt, so daß auch die erhaltenen Olefine keine einheitliche C-Zahl aufweisen. Außerdem kommen in diesen Olefin-Gemischen natürlich alle denkbaren isomeren Formen vor.

Neben den reinen Monoolefinen mit 6 bis 20 C-Atomen und Mischungen derselben können bei dem erfindungsgemäßen Verfahren auch solche mit einem Gehalt an Paraffinen eingesetzt werden. Der Paraffingehalt rührt daher, daß bei der Olefinherstellung keine vollständigen Umsätze erreicht und die nicht umgesetzten Paraffine nicht oder nicht vollständig abgetrennt werden. So enthält z.B. ein Olefin-Paraffin-Gemisch, das durch Chlorierung-Dehydrochlorierung gewonnen wird, ca. 70 Gewichtsprozent Paraffin.

Das eingesetzte Kohlenmonoxid kann durch bekannte Abtrennungsverfahren (Tieftemperaturdestillation, Molekularsiebtrennung) aus Synthesegas gewonnen werden. Es ist dabei nicht erforderlich, den Wasserstoff quantitativ zu entfernen, da sich ein Wasserstoffgehalt, der ca. 10 Volumenprozent nicht übersteigt, erfahrungsgemäß günstig auf die Reaktionsgeschwindigkeit der Umsetzung auswirkt. Alle Verunreinigungen, die die Aktivität des Katalysatorsystems beeinträchtigen, müssen dagegen weitgehend entfernt werden.

Als Alkanole können bei dem erfindungsgemäßen Verfahren alle primären und sekundären mit einer oder mehreren Hydroxylfunktionen eingesetzt werden. Bevorzugte Alkanole sind solche mit einer C-Zahl bis einschließlich 4, d.h. Methanol, Ethanol, Propanol, Isopropanol, n-Butanol, iso-Butanol und Butanol-2. Sofern es für die Aufarbeitung des Reaktionsgemisches nicht zweckmäßig ist, ein höheres Alkanol einzusetzen, wird als Veresterungskomponente bevorzugt Methanol verwendet.

Der verwendete Katalysator besteht aus einer Cobaltverbindung und einem Promotor. Geeignete Cobaltverbindungen sind Carbonyle, wie z.B. Dicobaltoctacarbonyl, carbonsaure Salze, wie z.B. Acetat, Naphthenat, 2-Ethylhexanoat und Stearat, weiterhin Carbonate und Oxide. Das Cobalt kann in diesen Verbindungen in allen möglichen Wertigkeitsstufen vorkommen. Als Promotoren kommen Pyridin sowie alle nicht ortho-substituierten halogenfreien Pyridinderivate, wie z.B. $\beta$- und $\gamma$-Picolin, 3,4- und 3,5-Lutidin und $\beta$- und $\gamma$-Ethylpyridin, infrage.

Das Promotor : Cobalt-Verhältnis liegt im Bereich von 3 bis 25 : 1. In diesem Verhältnis ist der Promotor in Molen angegeben und die Cobaltverbindung in Grammatom Cobalt. Innerhalb des angegebenen Bereichs von 3 bis 25 : 1 ist der Bereich von 5 bis 15 : 1 beim Einsatz von paraffinfreiem bzw. weitgehend paraffinfreiem Monoolefin (< 5 Gewichtsprozent Paraffin) und der Bereich von 10 bis 25 : 1 beim Einsatz von paraffinhaltigem Monoolefin (bis ca. 80 Gewichtsprozent Paraffin) bevorzugt. Beim Einsatz von Monoolefinen mit Paraffingehalten zwischen diesen Extremen verschiebt sich der optimale Bereich entsprechend. Dies gilt auch für die nachfolgenden optimalen Bereiche verfahrenskritischer Maßnahmen.

Die Cobaltkonzentration beträgt 0,02 bis 0,2 Grammatom Cobalt pro Mol Monoolefin. Dabei ist der Bereich von 0,02 bis 0,08 beim Einsatz von paraffinfreiem Monoolefin und der Bereich von 0,08 bis 0,2 beim Einsatz von paraffinhaltigem Monoolefin bevorzugt.

Das erfindungsgemäße Verfahren wird bei einer Temperatur von 165 bis 195, vorzugsweise 175 bis 185 °C, durchgeführt.

Der Druck liegt im Bereich von 150 bis 300, vorzugsweise 180 bis 270 bar.

Das molare Alkanol : Monoolefin-Verhältnis beträgt 1 bis 10 : 1. Der Bereich von 1 bis 4 : 1 ist dabei beim Einsatz von paraffinfreiem Monoolefin und der Bereich von 4 bis 10 : 1 beim Einsatz von paraffinhaltigem Monoolefin bevorzugt.

Die Verweilzeit, definiert als Quotient aus dem mit Flüssigkeit gefüllten Reaktorvolumen und der Summe der pro Zeiteinheit eingesetzten Volumina an bei 50 °C flüssigen Einsatzstoffen, ist beim erfindungsgemäßen Verfahren größer als 15 Minuten. Für paraffinfreies Monoolefin wählt man vorzugsweise eine Verweilzeit $\geq$ 30 Minuten und für paraffinhaltige Monoolefine eine solche von $\geq$ 60 Minuten. Es gibt keine kritische obere Grenze der Verweilzeit, vielmehr resultiert diese aus der bei gegebenem Reaktionsvolumen angestrebten Produktionshöhe pro Zeiteinheit.

Im allgemeinen wird das erfindungsgemäße Verfahren so durchgeführt, daß man das gegebenenfalls paraffinhaltige Monoolefin zusammen mit dem Alkanol, dem Promotor und der Cobaltverbindung in einem mit Rührer ausgerüsteten Autoklaven vorlegt und den Sauerstoff durch Spülen mit einem Inertgas entfernt. Nach dem Einstellen der gewünschten Reaktionstemperatur wird Kohlenmonoxid aufgepreßt. Durch eine automatisch gesteuerte Innenkühlung sowie durch wiederholtes Nachpressen von Kohlenmonoxid können sowohl Temperatur als auch Druck konstantgehalten werden. Setzt man als Cobaltverbindung ein Carbonyl ein, so empfiehlt es sich, bereits während der Aufheizphase einen die Stabilität des Carbonyls gewährleistenden Kohlenmonoxiddruck vorzulegen. Den Reaktionsverlauf kann man durch gaschromatographische Analyse von Proben, die während der Reaktion entnommen werden, verfolgen. Beim gewünschten Umsatz wird die Reaktion durch Abkühlen des Autoklaveninhalts und anschließendes Entspannen beendet.

Gemäß einer bevorzugten Ausführungsform der vorliegenden Erfindung wird nun das Reaktionsgemisch bei einer Temperatur von 20 bis 100, vorzugsweise 40 bis 60 °C, solange mit einem sauerstoffhaltigen Gas, vorzugsweise Luft, behandelt, bis die bei der destillativen Aufarbeitung zur Abscheidung von metallischem Cobalt führenden Cobaltverbindungen oxidativ zerstört sind. Die Behandlung kann man z.B. in einer Rieselsäule durchführen, indem man dem Reaktionsaustrag das sauerstoffhaltige Gas entgegenführt. Die oxidative Zerstörung läßt sich sehr einfach an der Farbänderung (von Braun-Orange nach Blau-Violett) erkennen.

Im Anschluß an die Behandlung mit einem sauerstoffhaltigen Gas wird der Reaktionsaustrag destillativ aufgearbeitet. Dabei kann man entweder so vorgehen, daß zunächst unumgesetztes Alkanol und Monoolefin sowie Promotor, gegebenenfalls Paraffin und Reaktionsprodukte vom cobalthaltigen Rückstand abgetrennt und dann einer fraktionierten Destillation unterworfen werden. Oder aber man führt die fraktionierte Destillation ohne vorheriges Abtrennen der Cobaltverbindungen durch. Alkanol, Monoolefin, Promotor und der cobalthaltige Rückstand können wieder in den Prozeß zurückgeführt werden.

Das erfindungsgemäße Verfahren kann sowohl diskontinuierlich als auch kontinuierlich durchgeführt werden. Bei der kontinuierlichen Verfahrensweise ist es in manchen Fällen angebracht, eine Katalysatorpräformierung vorzunehmen. Dabei geht man z.B. so vor, daß man die in Pyridin bzw. in Pyridinderivaten lösliche Cobaltverbindung in einer gesonderten Reaktionsstufe bei erhöhter Temperatur (120 bis 200 °C) und bei erhöhtem Druck (100 bis 300 bar) mit einem wasserstoffhaltigen Kohlenmonoxid (1 bis 60 Volumenprozent $H_2$) behandelt. Als Cobaltverbindung kann auch der durch destillative Aufarbeitung des Hydrocarboxilierungsgemisches erhaltene cobalthaltige Rückstand eingesetzt werden. Der präformierte Katalysator wird zusammen mit dem gegebenenfalls Paraffin enthaltenden Monoolefin und dem Alkanol in die Hydrocarboxilierungsstufe eingesetzt.

Die nach dem erfindungsgemäßen Verfahren hergestellten Alkylester gesättigter aliphatischer Carbonsäuren dienen als Ausgangsprodukte für Fettalkohole, Fettsäure und Fettamine sowie deren Derivate. Fettalkohole können dabei durch Hydrierung an Kupferchromitkatalysatoren, Fettsäuren durch Hydrolyse und Fettamine durch Umsetzung mit Ammoniak und anschließende Hydrierung aus den entsprechenden Estern gewonnen werden. Diese Produkte und ihre Derivate finden Anwendung als z.B. Waschmittelrohstoffe, Schmierölbestandteile, Emulgatoren und Weichmacher.

Die Erfindung wird durch die nachfolgenden Beispiele und Vergleichsbeispiele näher erläutert.


## Beispiel 1

In einem Autoklaven aus nichtrostendem Stahl werden 168 Gew.Teile eines statistischen Isomerengemisches innenständiger n-Dodecene mit einem $\alpha$-Olefin-Anteil < 1 Gew.-%, 64 Gew.Teile Methanol, 29,5 Gew.Teile Cobaltnaphthenat mit einem Gehalt von 10 Gew.% Cobalt und 46,5 Gew.Teile $\gamma$-Picolin vorgelegt. Aus den Einsatzmengen errechnet sich ein molares Verhältnis Methanol/Olefin = 2/1 ; Cobalt/Olefin = 0,05/1 und $\gamma$-Picolin/Cobalt = 10/1. Nach dem Aufheizen auf 180 °C werden zunächst über ein Dosiergefäß 2,5 bar $H_2$ aufgepreßt und dann wird durch Aufdrücken von Kohlenmonoxid (CO) ein Gesamtdruck von 180 bar eingestellt, der durch mehrmaliges Nachpressen von CO in einem Bereich ± 3 bar konstant gehalten wird. Die Reaktion wird durch Entnahme von Proben, die gaschromatografisch analysiert werden, während eines Zeitraumes von 6 Stunden verfolgt. Für einen Olefinumsatz von 60 Mol-% ergibt sich eine Raumzeitausbeute von 370 g Tridecansäuremethylester pro Stunde und pro Liter mit Flüssigkeit gefüllten Reaktorvolumens. Für 60 Mol-% Olefinumsatz beträgt die Selektivität bezüglich Tridecansäuremethylestern 95 Mol-%, der Anteil linearen Tridecansäuremethylesters (bezogen auf die Summe isomerer Tridecansäuremethylester) 74 Gew.-%.

### Beispiele 2 und 3

Der in Beispiel 1 beschriebene Versuch wird unter Beibehaltung der Reaktionsbedingungen und molaren Verhältnisse der Einsatzstoffe mit 8,55 Gew.Teilen $Co_2$ $(CO)_8$ bzw. 12,45 Gew.Teilen $Co(CH_3COO)_2 \cdot 4 H_2O$ als Katalysatorvorstufe anstelle von Co-naphthenat wiederholt.

### Tabelle I

| Beispiel | Katalysatorvorstufe | Raumzeitausbeute *) g/1·h | Esterselektivität *) Mol-% | linearer Esteranteil *) Gew.-% |
|---|---|---|---|---|
| 2 | $Co_2(CO)_8$ | 400 | 95 | 73 |
| 3 | $Co(CH_3COO)_2$ $4 H_2O$ | 360 | 94 | 74 |

*) für 60 Mol-% Olefinumsatz

### Beispiel 4

Der in Beispiel 1 beschriebene Versuch wird unter Beibehaltung der Reaktionsbedingungen und molaren Verhältnisse der Einsatzstoffe mit 39,5 Gew.Teilen Pyridin anstelle von γ-Picolin wiederholt. Für einen Olefinumsatz von 60 Mol-% ergibt sich eine Raumzeitausbeute von 320 g Tridecansäuremethylester pro Stunde und pro Liter mit Flüssigkeit gefüllten Reaktorvolumens ; die Selektivität beträgt 94 Mol-%, der Anteil linearer Ester 72 Gew.-%.

### Beispiel 5

In einem Autoklaven aus nichtrostendem Stahl werden 593 Gew.Teile eines Gemisches aus 425 Gew.-Teilen n-Dodecan und aus 168 Gew.Teilen eines statistischen Isomerengemisches innenständiger n-Dodecene mit einem α-Olefin-Anteil < 1 Gew.-%, 256 Gew.Teile Methanol, 82,5 Gew.Teile Cobaltnaphthenat mit einem Gehalt von 10 Gew.% Cobalt und 130 Gew. Teile γ-Picolin vorgelegt. Aus den Einsatzmengen errechnet sich ein molares Verhältnis Methanol/Olefin 8/1 ; Cobalt/Olefin = 0,14/1 und γ-Picolin/Cobalt = 10/1. Nach dem Aufheizen auf 180 °C werden zunächst über ein Dosiergefäß 3,5 bar $H_2$ aufgepreßt und dann wird durch Aufdrücken von CO ein Gesamtdruck von 250 bar eingestellt, der durch mehrmaliges Nachpressen von CO in einem Bereich ± 5 bar konstant gehalten wird. Die Reaktion wird durch die Entnahme von Proben, die gaschromatografisch analysiert werden, während eines Zeitraumes von 6 Stunden verfolgt. Für einen Olefinumsatz von 60 Mol-% ergibt sich eine Raumzeitausbeute von 100 g Tridecansäuremethylester pro Stunde und pro Liter mit Flüssigkeit gefüllten Reaktorvolumens. Für 60 Mol-% Olefinumsatz beträgt die Selektivität bezüglich Tridecansäuremethylester 96 Mol-%, der Anteil linearen Tridecansäuremethylesters (bezogen auf die Summe isomerer Tridecansäuremethylester) 75 Gew.-%.

### Beispiele 6 und 7

Der in Beispiel 5 beschriebene Versuch wird unter Beibehaltung der Reaktionsbedingungen und molaren Verhältnisse der Einsatzstoffe wiederholt mit der Ausnahme, daß das in Beispiel 5 eingesetzte Paraffin-Olefin-Gemisch durch folgende Gemische ersetzt wird.
Beispiel 6 :
14 Gew.Teile statistisches n-Decenisomerengemisch (α-Anteil < 1 Gew.-%)
138,6 Gew.Teile statistisches n-Undecenisemerengemisch (α-Anteil < 1 Gew.-%)
36 Gew.Teile Decan

356,5 Gew.Teile Undecan

Beispiel 7 :

134,4 Gew.Teile statistisches n-Dodecenisomerengemisch (α-Anteil < 1 Gew.-%)

36,4 Gew.Teile statistisches n-Tridecenisomerengemisch (α-Anteil < 1 Gew.-%)

345,5 Gew.Teile Dodecan

93,5 Gew.Teile Tridecan

Tabelle II

| Bei-spiel | C-Zahl des Olefin-Paraffin-Gemisches | Raumzeit-ausbeute [*] g/1·h | Esterselek-tivität [*] Mol-% | linearer Esterant. [*] Mol-% |
|---|---|---|---|---|
| 6 | $C_{10,11}$ | 110 | 96 | 76 |
| 7 | $C_{12,13}$ | 95 | 96 | 74 |

[*] für einen Umsatz von 60 Mol-% Olefin.

Beispiele 8 bis 12

Der in Beispiel 5 beschriebene Versuch wird unter Beibehaltung der Reaktionsbedingungen und molaren Verhältnisse der Einsatzstoffe wiederholt mit der Ausnahme, daß Methanol durch äquimolare Mengen höherer Alkanole ersetzt wird. Die für einen Olefinumsatz von 60 Mol-% erhaltenen Ergebnisse sind in der folgenden Tabelle aufgeführt.

Tabelle III

| Bei-spiel | Alkanol | Raumzeit-ausbeute Mol Ester/1·h | Esterselek-tivität Mol-% | linearer Esteranteil Gew.-% |
|---|---|---|---|---|
| 8 | Ethanol | 0,32 | 96 | 74 |
| 9 | Propanol | 0,21 | 95 | 74 |
| 10 | Propanol-(2) | 0,24 | 95 | 76 |
| 11 | Butanol | 0,19 | 96 | 73 |
| 12 | 2-Methylpro-panol | 0,16 | 95 | 72 |

Beispiel 13

Kontinuierliche Durchführung der Hydrocarboxylierungsreaktion mit gesonderter Katalysatorpräformierung :

a) Katalysatorpräformierung :

Ein Gemisch aus 29,5 Gew.Teilen Cobaltnaphthenat mit einem Gehalt von 10 Gew.% Cobalt und 46,5 Gew.Teilen γ-Picolin wird in einem Autoklaven mit Synthesegas (mit gleichen Volumenanteilen $H_2$ und CO) bei einer Temperatur von 170 °C und einem Druck von 160 bar kontinuierlich mit einer mittleren Verweilzeit von 0,5 Stunden umgesetzt.

b) Hydrocarboxylierung :

Das aus 29,5 Gew.Teilen Cobaltnaphthenat mit einem Gehalt von 10 Gew.% Cobalt und 46,5 Gew.-Teilen γ-Picolin wie unter a) beschrieben hergestellte Katalysatorsystem wird mit 168 Gew.Teilen eines statistischen Isomerengemisches innenständiger n-Dodecene (α-Anteil < 1 Gew.-%) und 64 Gew.Teilen Methanol mit Kohlenmonoxid bei einer Temperatur von 180 °C und einem Druck von 200 bar mit einer mittleren Verweilzeit von 1,2 Stunden umgesetzt. (Aus den Einsatzmengen errechnet sich ein molares Verhältnis Methanol/Olefin 2/1 ; Cobalt/Olefin = 0,05/1 und γ-Picolin/Cobalt = 10/1.) Auf diese Weise wird ein Olefinumsatz von 57 Mol-% erzielt. Das mit einer Selektivität von 97 Mol-% gebildete Gemisch isomerer Tridecansäuremethylester hat einen n-Anteil von 74 Gew.-%.

5

### Beispiel 14

Der in Beispiel 13 beschriebene Versuch wird unter Beibehaltung der Reaktionsbedingungen und molaren Verhältnisse der Einsatzstoffe wiederholt mit der Ausnahme, daß keine Katalysatorpräformierung durchgeführt wird. (Die Reaktionstemperatur in der Präformierstufe wird auf 20 °C gesenkt). Auf diese Weise wird ein Olefinumsatz von 51 Mol-% erzielt. Das mit einer Selektivität von 98 Mol-% gebildete Gemisch isomerer Tridecansäuremethylester hat einen n-Anteil von 73 Gew.-%.

### Beispiel 15

Kontinuierliche Durchführung der Hydrocarboxylierungsreaktion mit gesonderter Katalysatorpräformierung :

a) Katalysatorpräformierung :

Ein Gemisch aus 100,3 Gew.Teilen Cobaltnaphthenat mit einem Gehalt von 10 Gew.% Cobalt und 158 Gew.Teilen γ-Picolin wird in den unter 13 a) beschriebenen Bedingungen mit Synthesegas umgesetzt.

b) Hydrocarboxylierung :

Das aus 100,3 Gew.Teilen Cobaltnaphthenat mit einem Gehalt von 10 Gew.% Cobalt und 158 Gew.-Teilen γ-Picolin wie unter a) beschrieben hergestellte Katalysatorsystem wird mit 609,8 Gew.Teilen eines Gemisches aus

134,4 Gew.Teilen statistischem n-Dodecenisomerengemisch (α-Anteil < 1Gew.-%)

36,4 Gew.Teilen statistischem n-Tridecenisomerengemisch (α-Anteil < 1 Gew.-%)

345,5 Gew.Teilen Dodecan

93,5 Gew.Teilen Tridecan

und 256 Gew.Teilen Methanol mit Kohlenmonoxid bei einer Temperatur von 180 °C und einem Druck von 250 bar mit einer mittleren Verweilzeit von 2 Stunden umgesetzt. (Aus den Einsatzmengen errechnet sich ein molares Verhältnis Methanol/Olefin = 8,1 ; Cobalt/Olefin = 0,17/1 und γ-Picolin/Cobalt = 10/1). Auf diese Weise wird ein Olefinumsatz von 62 Mol-% erzielt. Das mit einer Selektivität von 95 Mol-% gebildete Gemisch isomerer Tridecan- und Tetradecansäuremethylester hat einen n-Anteil von 75 Gew.-%.

c) Aufarbeitung :

1 000 g/h des nach b) erhaltenen Reaktionsgemisches werden bei einer Temperatur von 55 °C in einer mit Raschigringen gefüllten Rieselsäule (Innendurchmesser : 2 cm ; Länge : 100 cm) im Gegenstrom mit 50 l Luft/h behandelt.

Das oxidativ nachbehandelte Reaktionsgemisch wird einer fraktionierten Destillation unterworfen. Nach Abtrennung von Methanol, γ-Picolin, unumgesetztem Olefin, Paraffin sowie Tridecan- und Tetradecansäuremethylestern erhält man 105 g eines cobalt-haltigen Rückstandes, der nach Auflösen z.B. in γ-Picolin wieder als Katalysatorvorstufe in die Reaktion zurückgeführt werden kann.

### Vergleichsbeispiele A und B

Der in Beispiel 1 beschriebene Versuch wird unter Beibehaltung der Reaktionsbedingungen und molaren Verhältnisse der Einsatzstoffe wiederholt, mit der Ausnahme, daß andere Verhältnisse von γ-Picolin/Cobalt gewählt werden. Die für einen Olefinumsatz von 60 Mol-% erhaltenen Ergebnisse sind in Tabelle 4 aufgeführt.

### Vergleichsbeispiel C

Der in Vergleichsbeispiel B beschriebene Versuch wird unter Beibehaltung der Reaktionsbedingungen und molaren Verhältnisse der Einsatzstoffe wiederholt, mit der Ausnahme, daß γ-Picolin durch eine äquimolare Menge Pyridin ersetzt wird. Die für einen Olefinumsatz von 60 Mol-% erhaltenen Ergebnisse sind in Tabelle 4 aufgeführt.

### Tabelle IV

| Vergl. Beispiel | Promotor | Prom./ Co-Verhältnis | Raumzeitausbeute g/l·h | Esterselektivität Mol-% | linearer Esterant. Gew.-% |
|---|---|---|---|---|---|
| A | γ-Picolin | 2,5 | 60 | 80 | 65 |
| B | γ-Picolin | 35 | 40 | 95 | 60 |
| C | Pyridin | 35 | 80 | 95 | 66 |

### Vergleichsbeispiele D und E

Der in Beispiel 1 beschriebene Versuch wird unter Beibehaltung der molaren Verhältnisse der Einsatzstoffe und der Reaktionsbedingungen mit Ausnahme der Reaktionstemperatur wiederholt. Die für einen Olefinumsatz von 60 Mol-% erhaltenen Ergebnisse sind in der nachfolgenden Tabelle aufgeführt.

Tabelle V

| Vergl. Bei- spiel | Reaktion- temperatur $^o$C | Raumzeit- ausbeute g/l·h | Esterselek- tivität Mol-% | linearer Ester- anteil Gew.-% |
|---|---|---|---|---|
| D | 150 | 40 | .97 | 52 · |
| E | 200 | 10 | 81 | 70 |

### Vergleichsbeispiele F und G

Der in Beispiel 1 beschriebene Versuch wird unter Beibehaltung der molaren Verhältnisse der Einsatzstoffe und der Reaktionsbedingungen mit Ausnahme des Gesamtdruckes wiederholt. Die für einen Olefinumsatz von 60 Mol-% erhaltenen Ergebnisse sind in der nachfolgenden Tabelle aufgeführt.

Tabelle VI

| Vergl. Bei- spiel | Gesamt- druck bar | Raumzeit- ausbeute g/l·h | Esterselek- tivität Mol-% | linearer Ester- anteil Gew.-% |
|---|---|---|---|---|
| F | 60 | 50 | 93 | 78 |
| G | 350 | 300 | 95 | 55 |

### Vergleichsbeispiel H

Der in Beispiel 5 beschriebene Versuch wird unter Beibehaltung der Reaktionsbedingungen und der molaren Verhältnisse der Einsatzstoffe wiederholt mit der Ausnahme, daß er bei einem Gesamtdruck von 200 bar (anstelle von 250 bar), mit einer äquimolaren Menge Pyridin (anstelle von γ-Picolin) und einem molaren Verhältnis Methanol/Olefin = 15/1 (anstelle von 8/1) durchgeführt wird. Die für einen Olefinumsatz von 60 Mol-% erhaltenen Ergebnisse sind in Tabelle 7 aufgeführt.

Tabelle VII

| Vergl. Bei- spiel | Methanol/ Olefin- Verhältnis | Raumzeit- ausbeute g/l.h | Esterselek- tivität Mol-% | linearer Ester anteil Gew.-% |
|---|---|---|---|---|
| H | 15/1 | 25 | 94 | 71 |

### Vergleichsbeispiel J

Der in Beispiel 15 beschriebene Versuch wird unter Beibehaltung der Reaktionsbedingungen und der molaren Verhältnissse der Einsatzstoffe wiederholt mit der Ausnahme, daß unter Beibehaltung des Verhältnisses von γ-Picolin/Cobalt = 10 ein Verhältnis Cobalt/Olefin von 0,01/1 (anstelle von 0,17/1) gewählt wird. Auf diese Weise wird ein Olefinumsatz von 20 Mol-% erzielt. Das mit einer Selektivität von 92 Mol-% gebildete Gemisch isomerer Tridecan- und Tetradecansäuremethylester hat einen n-Anteil von 78 Gew.-%.

### Vergleichsbeispiel K

Der in Beispiel 13 beschriebene Versuch wird unter Beibehaltung der Reaktionsbedingungen und der molaren Verhältnisse der Einsatzstoffe wiederholt, mit der Ausnahme, daß unter Beibehaltung des Verhältnisses von γ-Picolin/Cobalt = 10 ein Verhältnis. Cobalt/Olefin von 0,015/1 (anstelle von 0,05/1) gewählt wird. Auf diese Weise wird ein Olefinumsatz von 25 Mol-% erzielt. Das mit einer Selektivität von 92 Mol-% gebildete Gemisch isomerer Tridecansäuremethylester hat einen n-Anteil von 77 Gew.-%.

Vergleichsbeispiel L

Der in Beispiel 13 beschriebene Versuch wird unter Beibehaltung der Reaktionsbedingungen und der molaren Verhältnisse der Einsatzstoffe wiederholt, mit der Ausnahme, daß eine mittlere Verweilzeit von 0,25 Stunden in der Hydrocarboxylierungsstufe gewählt wird. Auf diese Weise wird ein Umsatz von 25 Mol-% erzielt. Das mit einer Selektivität von 93 Mol-% gebildete Gemisch isomerer Tridecansäuremethylester hat einen n-Anteil von 77 Gew.-%.

**Ansprüche**

1. Verfahren zur Herstellung von Alkylestern gesättigter aliphatischer Carbonsäuren durch Umsetzung innenständige Doppelbindungen enthaltender aliphatischer Monoolefine mit Kohlenmonoxid und Alkanol in Gegenwart eines Katalysators bestehend aus einer Cobaltverbindung und einem Promotor aus der Gruppe Pyridin und/oder nicht-ortho-substituierter Alkylpyridine bei erhöhtem Druck und erhöhter Temperatur, dadurch gekennzeichnet, daß man gegebenenfalls Paraffine enthaltende Monoolefine mit 6 bis 20 C-Atomen bei
   a) einem Promotor : Cobalt-Verhältnis von 3 bis 25 : 1,
   b) einer Cobaltkonzentration von 0,02 bis 0,2 Grammatom Cobalt pro Mol Monoolefin,
   c) einer Temperatur von 165 bis 195 °C,
   d) einem Druck von 150 bis 300 bar,
   e) einem molaren Alkanol : Monoolefin-Verhältnis von 1 bis 10 : 1 und
   f) einer Verweilzeit von mehr als 15 Minuten umsetzt.
2. Verfahren nach Anspruch 1, dadurch gekennzeichnet, daß man ein Gemisch überwiegend innenständige Doppelbindungen enthaltender aliphatischer Monoolefine einsetzt.
3. Verfahren nach Anspruch 2, dadurch gekennzeichnet, daß man als überwiegend innenständige Doppelbindungen enthaltendes Gemisch aliphatischer Monoolefine ein solches einsetzt, das durch Chlorierung von Paraffinen und anschließende Dehydrochlorierung der erhaltenen Chlorparaffine gewonnen wird.
4. Verfahren nach den Ansprüchen 1 bis 3, dadurch gekennzeichnet, daß man als Alkanol Methanol einsetzt.
5. Verfahren nach den Ansprüchen 1 bis 4, dadurch gekennzeichnet, daß man das Reaktionsgemisch nach beendeter Umsetzung bei Temperaturen von 20 bis 100 °C mit einem sauerstoffhaltigen Gas behandelt, die unumgesetzten Reaktanten, den Promotor und die Reaktionsprodukte destillativ abtrennt und den cobalthaltigen Rückstand zurückführt.

**Claims**

1. A process for the preparation of an alkyl ester of a saturated aliphatic carboxylic acid by reaction of an aliphatic monoolefin containing a non-terminal double bond with carbon monoxide and an alkanol at elevated pressure and temperature in the presence of a catalyst comprising a cobalt compound and a promoter selected from pyridine and alkylpyridines not substituted in the ortho position, characterised in that a mono-olefin of 6 to 20 carbon atoms, which optionally contains a paraffin, is reacted at
   a) a promoter : cobalt ratio of 3 : 1 to 25 : 1,
   b) a cobalt concentration of 0.02 to 0.2 gram atoms of cobalt per mole of mono-olefin,
   c) a temperature of 165 to 195 °C,
   d) a pressure of 150 to 300 bars,
   e) a molar ratio alkanol : mono-olefin of 1: 1 to 10: 1, and
   f) a residence time of more than 15 minutes.
2. A process according to claim 1, characterised in that a mixture composed predominantly of an aliphatic mono-olefin containing non-terminal double bonds is used.
3. A process according to claim 2, characterised in that the mixture composed predominantly of an aliphatic mono-olefin containing non-terminal double bonds used has been obtained by chlorination of a paraffin and subsequent dehydrochlorination of the resulting chloroparaffin.
4. A process according to any of claims 1 to 3, characterised in that methanol is used as the alkanol.
5. A process according to any of claims 1 to 4, characterised in that after the end of the reaction the reaction mixture is treated with an oxygen-containing gas at a temperature of 20 to 100 °C, the unconverted reactants the promoter and the reaction products are separated by distillation and the cobalt-containing residue is recycled.

**Revendications**

1. Procédé de préparation d'esters alkyliques d'acides carboxyliques aliphatiques saturés par réaction de mono-oléfines aliphatiques, contenant des doubles liaisons non terminales, sur le monoxyde de carbone et un alcanol en présence d'un catalyseur formé d'un composé de cobalt et d'un adjuvant du groupe qui comprend la pyridine et/ou les alkylpyridines non substituées en ortho, à pression élevée et à température élevée, caractérisé par le fait que l'on fait réagir des mono-oléfines comportant de 6 à 20 atomes de carbone, contenant éventuellement des paraffines, dans les conditions suivantes :
   a) un rapport adjuvant : cobalt compris entre 3: 1 et 25: 1,
   b) une concentration de cobalt de 0,02 à 0,2 atome-gramme de cobalt par mole de mono-oléfine,
   c) une température de 165 à 195 °C,
   d) une pression de 150 à 300 bar,
   e) un rapport molaire alcanol : mono-oléfine compris entre 1: 1 et 10: 1 et
   f) un temps de séjour supérieur à 15 minutes.

2. Procédé selon la revendication 1, caractérisé par le fait que l'on introduit un mélange de mono-oléfines aliphatiques contenant en majorité des doubles liaisons non terminales.

3. Procédé selon la revendication 2, caractérisé par le fait que comme mélange de mono-oléfines aliphatiques contenant en majorité des doubles liaisons non terminales, on introduit un mélange que l'on obtient par chloration de paraffines et ensuite par déchlorhydration des chloroparaffines obtenues.

4. Procédé selon les revendications 1 à 3, caractérisé par le fait que, comme alcanol, on introduit le méthanol.

5. Procédé selon les revendications 1 à 4, caractérisé par le fait qu'après la fin de la réaction, on traite le mélange réactionnel, à des températures de 20 à 100 °C, par un gaz oxygéné que l'on sépare par distillation les réactifs non convertis, l'adjuvant et les produits de réaction et que l'on recycle le résidu contenant du cobalt.

9